(19) Europäisches Patentamt
European Patent Office
Office européen des brevets

(11) Veröffentlichungsnummer : **0 051 235**
**B1**

(12) # EUROPÄISCHE PATENTSCHRIFT

(45) Veröffentlichungstag der Patentschrift : **09.01.85**

(51) Int. Cl.[4] : **C 07 C 43/275**, C 07 C 41/16

(21) Anmeldenummer : **81108876.4**

(22) Anmeldetag : **24.10.81**

(54) **Verfahren zur Herstellung von Diphenyläthern.**

(30) Priorität : **30.10.80 DE 3040849**

(43) Veröffentlichungstag der Anmeldung : **12.05.82 Patentblatt 82/19**

(45) Bekanntmachung des Hinweises auf die Patenterteilung : **09.01.85 Patentblatt 85/02**

(84) Benannte Vertragsstaaten : **BE CH DE FR GB IT LI NL**

(56) Entgegenhaltungen :
**EP-A- 0 008 734**
**EP-A- 0 024 019**
**DE-A- 1 930 449**
**DE-A- 2 228 609**
**DE-A- 2 242 519**
**FR-A- 2 460 283**
**Krauch, Kunz, Reaktionen der organischen Chemie, Dr. Alfred Hüthig Verlag, Heidelberg 1976, Seite 320**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Kuckertz, Herbert, Dr.**
**Ludwig-Schäfer-Weg 11**
**D-6233 Kelkheim (Taunus) (DE)**
Erfinder : **Schaeffer, Georg, Dr.**
**Herderstrasse 58**
**D-6238 Hofheim am Taunus (DE)**

**EP 0 051 235 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Diaryläther — und hiervon hauptsächlich die Diphenyläther — sind vornehmlich Zwischenprodukte auf verschiedenen Sachgebieten wie z. B. auf dem Pharma- und Pflanzenschutzsektor. Der Diphenyläther m-Phenoxytoluol beispielsweise ist Zwischenprodukt für die Herstellung von Insektiziden vom Pyrethroid-Typ, die sich durch hohe insektizide Wirksamkeit bei geringer Toxizität gegenüber Warmblütlern auszeichnen.

Zur Herstellung der Diaryläther ist eine Reihe verschiedener Methoden bekannt. Eine vorteilhafte und gängige derartige Methode ist die sog. Ullmann-Reaktion. Diese besteht in der Umsetzung von Alkaliphenolaten mit Arylhalogeniden in Gegenwart von Kupfer oder von Kupferverbindungen als Katalysatoren bei erhöhter Temperatur (vgl. Krauch-Kunz, « Reaktionen der organischen Chemie », D. Hüthig Verlag GmbH, Heidelberg, 1976, S. 320).

Die Ullmann-Reaktion ist ihrerseits wiederum in einer Reihe verschiedener Ausführungsformen bekannt (Variation der Ausgangsmaterialien, der Cu-Katalysatoren, der Reaktionstemperatur, der Lösungsmittel etc.). Nach Houben-Weyl, « Methoden der organischen Chemie », G. Thieme Verlag, Stuttgart, 1965, Band VI/3, S. 86 reagieren die Bromverbindungen (Arylbromide) leichter als die Chlorverbindungen. Nur wenn der Arylkern noch bestimmte aktivierende Gruppe enthält, sollen auch die Arylchloride hinreichend reaktionsfähig sein. Nach der in dieser Literaturstelle beschriebenen Verfahrensvorschrift wird bei der Ullmann-Reaktion als Katalysator Cu-Pulver verwendet. Die Reaktionstemperatur ist zwischen 150 und 230 °C angegeben. Für verschiedene Fälle wird überschüssiges Phenol oder auch Dimethylformamid als Lösungs- oder Verdünnungsmittel empfohlen. Zum Beispiel aus K-p-Kresolat und 4-Br-Toluol soll so in Gegenwart von Cu-Bronze als Katalysator sowie auch von freiem p-Kresol bei etwa 200 bis 240 °C in etwa 2 Stunden Bis-[4-Methylphenyl]-äther in einer Ausbeute von 87 % d. Th. erhalten werden.

In Beispiel 1 der DE-OS 22 28 609 wird für die Ullmann-Reaktion des K-Salzes des Biphenyl-4-ols mit p-Chlortoluol ein aus CuCl, $CuCl_2$, $CuCO_3 \cdot Cu(OH)_2 \cdot H_2O$, Cu-Pulver und aktivieirtem $Al_2O_3$ bestehendes Katalysatorgemisch verwendet. Bei Temperaturen bis 230 °C wurden so in einer Reaktionszeit von etwa 30 Stunden 76 % d. Th. an p-Biphenylyloxytoluol erhalten.

In der DE-OS 22 42 519 sind für die dort beschriebene Ausführungsart der Ullmann-Reaktion verschiedene Cu-Verbindungen als Katalysatoren (CuO, $Cu_2O$, $CuCO_3$, CuCl, $CuCl_2$, CuBr etc.) — darunter auch basisches Cu-Carbonat — angegeben, wenngleich in sämtlichen Beispielen als Katalysator immer nur CuO verwendet wurde. Bei Reaktionstemperaturen zwischen 120 und 200 °C in Anwesenheit von bis zu 50 Mol % des entsprechenden (dem eingesetzten Alkaliphenolat zugrundeliegenden) freien Phenols sollen nach den Beispielen in einigen Stunden Reaktionszeit Ausbeuten an den jeweiligen Diphenyläthern zwischen etwa 60 und 75 % d. Th. erzielt werden. Vorzugsweise Cu-Pulver, jedoch auch Cu-Oxide, -carbonate, -chloride, -bromide und -sulfate werden als Katalysatoren für die Ullmann-Reaktion auch genannt in der FR-OS 78 16746 (Publikationsnummer 2392950). Nach der dort beschriebenen Reaktionsvariante sollen vor allem höhere Überschüsse an freiem (dem jeweiligen Alkaliphenolat zugrundeliegenden) Phenol verwendet werden.

Ähnlich arbeitet auch das Verfahren der BE-PS 874 981 zur Herstellung von m-Phenoxytoluol durch Ullmann-Reaktion von Alkali-m-kresolat mit Chlorbenzol in Gegenwart von CuCl als Katalysator (bei 150 bis 170 °C und überschüssigem m-Kresol).

Vornehmlich CuCl als Katalysator wird weiterhin verwendet bei der Ullmann-Reaktion von Alkaliphenolaten und halogenbenzolen (bei Temperaturen vorzugsweise zwischen etwa 130 und 180 °C in Gegenwart von überschüssigem Phenol) gemäß FR-A 2 460 283 ; andere $CU^{I}$-Verbindungen (vor allem CuBr, $CuOCOCH_3$, $Cu_2O$) sind als Katalysatoren noch namentlich erwähnt.

Ebenfalls CuCl ist bevorzugter Katalysator bei der Herstellung von Chlorderivaten von m-Diphenoxybenzol durch die Ullmann-Reaktion von Resorcin-Dialkalisalzen mit bestimmten Anteilen eines Monohalogenbenzols und eines m- oder p-Halogenchlorbenzols gemäß DE-A 1 930 449 ; auch hier sind noch verschiedene andere möglichen Cu-Katalysatoren (CuBr, $Cu_2SO_4$, $CuCl_2$, $CuBr_2$, $Cu(OCOCH_3)_2$ etc.) genannt.

Obwohl die bekannten Ausführungsformen der Ullmann-Reaktion zur Herstellung von Diaryläthern im allgemeinen recht befriedigend verlaufen, war es im Zuge der Bestrebungen nach immer weiterer Verfahrensoptimierung wünschenswert und bestand die Aufgabe, die Verfahren noch weiter zu verbessern und damit noch wirtschaftlicher zu gestalten.

Diese Aufgabe konnte erfindungsgemäß durch die Auswahl ganz spezieller Cu-Katalysatoren, nämlich spezieller basischer Cu-Carbonate, gelöst werden.

Erfindungsgegenstand ist daher ein Verfahren zur Herstellung von Diphenyläthern durch Umsetzung von unsubstituierten oder durch niedere Alkylgruppen, Halogen, CN, CHO und/oder COOR (R = niederes Alkyl) ein- oder mehrfach substituierten Alkaliphenolaten mit unsubstituierten oder durch die gleichen wie die bei den Alkaliphenolaten genannten Substituenten ein- oder mehrfach substituierten Halogenbenzolen in Gegenwart von Cu-Verbingunden als Katalysatoren bei erhöhter Temperatur, das dadurch gekennzeichnet ist, daß man als Cu-Verbindungen basische Cu-Carbonate der Formeln $2\,CuCO_3 \cdot Cu(OH)_2$, $CuCO_3 \cdot Cu(OH)_2 \cdot H_2O$ und/oder $CuCO_3 \cdot Cu(OH)_2 \cdot 1/2\ H_2O$ verwendet, wobei die letztgenannte Verbindung bevorzugt ist.

Die Katalysatoren können sowohl einzeln als auch in Mischung miteinander verwendet werden.

Der bevorzugte Mengenbereich liegt zwischen 0,0001 und 5 Mol-%, insbesondere zwischen 0,001 und 0,01 Mol-% bezogen auf das Alkaliphenolat.

Die erfindungsgemäß eingesetzten Katalysatoren besitzen bei der Ullmann-Reaktion insbesondere eine erheblich höhere katalytische Aktivität als die übrigen bekannten Cu-Katalysatoren, was bei gleichen molaren Katalysatormengen zu einer erheblichen Verkürzung der Reaktionszeit oder bei gleichen Katalysatormengen und Reaktionszeiten zu erhöhten Umsätzen und Ausbeuten führt.

Es war außerordentlich überraschend, daß die erfindungsgemäße Auswahl ein derart verbessertes Resultat liefert, weil aufgrund des Standes der Technik nicht zu erwarten war, daß in der Reihe der bekannten Cu-Katalysatoren für die Ullmann-Reaktion derartige Unterschiede bezüglich der katalytischen Aktivität auftreten können.

Als Ausgangs-Alkaliphenolate kommen für das erfindungsgemäße Verfahren im Prinzip alle möglichen Alkaliphenolate in Betracht. Bevorzugt sind die Na- und K-, insbesondere die K-Salze des unsubstituierten Phenols $C_6H_5OH$ selbst sowie des im Kern ein- oder mehrfach — insbesondere einfach — substituierten Phenols. Als Substituenten kommen hier bevorzugt folgende Gruppen infrage : niedere Alkylgruppen ($C_1$-$C_4$-Alkyl, vorzugsweise $CH_3$ und $C_2H_5$, insbesondere $CH_3$), Halogen (F, Cl, Br), CN, CHO, $CH(OR)_2$, COOR (R = niederes Alkyl).

Besonders bevorzugte Alkaliphenolate sind die Alkalisalze des unsubstituierten Phenols sowie des in o-, m- oder p-Stellung durch eine niedere Alkylgruppe oder Fluor einfach substituierten Phenols. Als Ausgangs-Halogenbenzole dienen sowohl das nicht weiter substituierte Monohalogenbenzol $C_6H_5Hal$ selbst (Hal = Cl oder Br, vorzugsweise Cl), als auch im Kern substituierte Halogenbenzole, wobei als Substituenten die gleichen wie auch für die Alkaliphenolate infrage kommen. Wenn die Substituenten ebenfalls Halogen (F, Cl, Br) sind, sind die ausgangshalogenbenzole natürlich keine Mono-, sondern Di- oder Polyhalogenbenzole. Es hängt dann von dem Molverhältnis Alkaliphenolat : Halogenbenzol ab, ob einer oder mehrere Halogensubstituenten mit dem Alkaliphenolat reagieren. Fluorsubstituenten reagieren bei dieser Reaktion allerdings nicht oder nur in untergeordnetem Maß. Bevorzugte Halogenbenzole sind die Mono- und Dichlorbenzole sowie die Mono- und Dibrombenzole und die durch eine niedere Alkylgruppe einfach substituierten Monochlor- und Monobrom-Benzole ; besonders bevorzugte Halogenbenzole sind Monochlor- und Monobrombenzol sowie die durch eine niedere Alkylgruppe einfach substituierten Monochlor- und Monobrombenzole, wobei jedoch die Chlorverbindungen ganz besonders bevorzugt sind.

Eine besonders bevorzugte Ausführungsform der Erfindung besteht weiterhin darin, daß einer der Reaktionspartner Alkaliphenolat und Halogenbenzol in durch eine niedere Alkylgruppe (einfach) substituierter Form und der andere Reaktionspartner in nicht weiter substituierter Form eingesetzt wird (also z. B. Umsetzung von Alkalikresolat plus Monochlorbenzol, oder von Alkaliphenolat plus Monochlortoluol).

Das erfindungsgemäße Verfahren wird ansonsten auf die für die Ullmann-Reaktion übliche Weise durchgeführt. In einer beispielhaften Durchführungsweise wird das Alkaliphenolat zunächst so hergestellt, indem man Alkalihydroxid (NaOH und/oder KOH, vorzugsweise KOH) in fester oder in Wasser gelöster Form mit dem jeweilige Phenol und dem Halogenbenzol zusammengibt. Die Entwässerung kann dann mittels eines Wasserauskreisers unter Rückfluß mit dem Halogenbenzol als Schleppmittel erfolgen. Zweckmäßigerweise verwendet man einen Überschuß an dem entsprechenden Phenol und Halogenbenzol, weil dadurch sowohl der Entwässerungsvorgang als auch die spätere Kondensationsreaktion erleichtert wird (schnellere und vollständige Umsetzung des Alkalihydroxids, leichtere Rührbarkeit und Filtrierbarkeit des Alkalihalogenid enthaltenden Reaktionsgemisches, keine Abscheidung von Alkaliphenolat etc.).

Das Molverhältnis von Alkalihydroxid zu dem entsprechenden Phenol zu Halogenbenzol wird dabei zweckmäßig zwischen etwa 1 : 1 : 1 und etwa 1 : 3 : 3 gewählt. Die Entwässerung erfolgt normalerweise unter Normaldruck. Allerdings wird man bei Verwendung hochsiedender Halogenbenzole entweder unter Vakuum entwässern oder aber unter Zusatz eines weiteren Schleppmittels wie z. B. von Toluol oder Xylol bei Normaldruck.

Die Zugabe des Katalysators erfolgt normalerweise nach der Entwässerung, wenngleich die Katalysatorzugabe auch vor der Entwässerung möglich ist.

Beispielsweise nach der Zugabe des Katalysators hält man das Reaktionsgemisch vorteilhaft unter Rühren bei Temperaturen zwischen etwa 50 und 200 °C, vorzugsweise zwischen etwa 100 und 200 °C, insbesondere zwischen etwa 130 und 170 °C, einige Stunden (manchmal auch nur einige Minuten) lang, bis z. B. durch Messung des Alkaligehaltes festgestellt wird, daß die Umsetzung vollständig abgelaufen ist. Während dieser Periode geht die ursprüngliche Lösung durch die Abscheidung von Alkalihalogenid in eine Suspension über.

Nach Beendigung der Reaktion kann das Reaktionsgemisch nach verschiedenen Methoden aufgearbeitet werden. Man kann z. B. das Alkalihalogenid und den Cu-Katalysator durch Filtration abtrennen und das Filtrat durch fraktionierte Destillation aufarbeiten. Das dann als Vorlauf anfallende Gemisch aus entsprechendem Phenol und Halogenbenzol läßt sich ohne Trennung in die Komponenten wieder bei Folgeansätzen verwenden.

Eine andere Aufarbeitungstechnik besteht z. B. darin, das Reaktionsgemisch mit Wasser bzw. verdünnter wäßriger Säure zu versetzen und somit das Alkalihalogenid und den Katalysator gelöst in der

sich dann abscheidenden wäßrigen Phase abzutrennen. Die weitere Aufarbeitung der organischen Phase kann dann wie das vorher beschriebene Filtrat durch Fraktionierung erfolgen.

Es ist möglich, das Verfahren sowohl diskontinuierlich als auch kontinuierlich durchzuführen.

Die erfindungsgemäße Variante der an sich bekannten Ullmann-Reaktion liefert die entsprechenden (gegebenenfalls substituierten) Diphenyläther in hohen Ausbeuten und — bei molmäßig gleichen Katalysatormengen wie bei den bekannten Ausführungsarten — in dem gegenüber kürzeren Reaktionszeiten. Die Erfindung stellt daher einen erheblichen Fortschritt dar.

Die folgenden Beispiele sollen nun der weiteren Erläuterung der Erfindung dienen.

Beispiel 1 enthält einige Vergleichsversuche mit gleichen molaren Mengen (gleicher Cu-Gehalt) anderer (als der erfindungsgemäß verwendeten) Cu-Katalysatoren; aus diesem Beispiel und den Vergleichsbeispielen geht die überlegene katalytische Aktivität der erfindungsgemäß verwendeten Katalysatoren deutlich hervor.

Beispiel 1 mit Vergleichsbeispielen:

Bei den Versuchen a) bis b) wurden in einem 1 l Vierhalskolben jeweils vorgelegt:

63,75 g 88-%ige feste KOH→1 Mol.

Dazu wurden in $N_2$-Atmosphäre unter Rühren gegeben

324 g m-Kresol→3 Mol und
338 g Chlorbenzol→3 Mol.

Das Gemisch wurde mittels eines Wasserauskreisers am Rückfluß bei Normaldruck entwässert. Es schieden sich jeweils ca. 26 g wäßrige Phase ab. Am Ende der Entwässerung wurde der Wasserauskreiser durch einen Rückflußkühler ersetzt und die organische Phase im Wasserauskreiser in den Reaktionskolben zurückgegeben. Zu der entwässerten Lösung wurden jeweils 0,01 Mol der in der nachstehenden Tabelle aufgeführten Kupfersalze gegeben. Die 0,01 molare Kupfersalzmenge ist dabei stets auf den Kupfergehalt (gleicher Cu-Gehalt!) bezogen.

Nach 4-stündiger Reaktion am Rückfluß bei Normaldruck, d. h. bei ca. 140 °C, wurde der Gehalt an 3-Phenoxytoluol gas-chromatographisch und der Umsatz des K-m-Kresolats titrimetrisch mit 0,1 n Salzsäure ermittelt.

Es ergaben sich die in der Tabelle aufgeführten K-m-Kresolat-Umsätze zu 3-Phenoxytoluol in über 98 %-iger Selektivität.

Daraus geht hervor, daß $Cu(CO_3) \cdot Cu(OH)_2 \cdot 0{,}5\ H_2O$ nach 4 Stunden Reaktionszeit einen 2,76 mal höheren Umsatz erbringen als die anderen Cu-Katalysatoren.

Tabelle

| Versuch | Katalysator | (gr) | % Umsatz KOH |
|---|---|---|---|
| a) * | $CuCO_3 \cdot Cu(OH)_2 \cdot 0{,}5\ H_2O$ | 1,1 | 69 |
| b) ** | Mischkatalysator aus | | |
| | CuCl | 0,44 | |
| | $CuCl_2 \cdot 2\ H_2O$ | 0,22 | |
| | $CuCO_3\ Cu(OH)_2 \cdot 0{,}5\ H_2O$ | 0,16 | 25 |
| | Cu-Pulver | 0,17 | |
| | $Al_2O_3$ (aktiviert) | 1,05 | |
| c) ** | CuCl | 0,99 | 25 |
| d) ** | $CuCl_2 \cdot 2\ H_2O$ | 1,7 | 23 |
| e) ** | CuO | 0,79 | 9 |
| f) ** | Cu-Pulver | 0,63 | 4 |
| g) ** | Cu $SO_4$ | 1,59 | 2 |
| h) ** | ohne Katalysator | 0 | 0 |

* erfindungsgemäß.
** Vergleich.

Aus dem folgenden Beispiel 2 geht hervor, daß unter ähnlichen Reaktionsbedingungen bei Verlängerung der Reaktionszeit auf 6 Stunden der erfindungsgemäß verwendete Katalysator zu praktisch quantitativem K-m-Kresolat-Umsatz bei praktisch quantitativer Selektivität zu 3-Phenoxytoluol führt.

Beispiel 2

3-Phenoxytoluol aus K-m-Kresolat und Chlorbenzol

In einem 4-l-Glas-Vierhalskolben werden vorgelegt :

224,4 g festes KOH 88 %ig → 3,52 Mol.

Dazu wurden in $N_2$-Atmosphäre unter Rühren gegeben

1 298 g m-Kresol → 12 Mol und
1 351 g Chlorbenzol → 12 Mol.

Während deren Zugabe stieg die Temperatur von ca. 20 °C auf 50 bis 60 °C an.

Das Gemisch wurde mittels eines Wasserabscheiders (Wasser oben), der vorher mit 200 g Chlorbenzol gefüllt worden war, am Rückfluß bei einer Sumpftemperatur von 120-136 °C während 1 1/2 Stunden entwässert.

In das dann auf 130 °C abgekühlte Gemisch wurden 4 g pulverförmiges $CuCO_3 \cdot Cu(OH)_2 \cdot 1/2\ H_2O$ (= 0,017 Mol) gegeben. Anschließend wurde 6 Stunden lang bei 137-142 °C unter $N_2$ und Rühren am Rückfluß erhitzt. Danach wurde der Wasserabscheider, einschließlich der darin enthaltenen 89 g wäßriger Phase sowie 180 g organische Phase (Chlorbenzol) entfernt.

In das im Reaktionskolben verbliebene Gemisch (2 815 g) wurden nach der Abkühlung auf 40 °C 1 000 ml Wasser gegeben. Mit Salzsäure wurde zunächst pH 7 eingestellt (0,273 HCl) und schließlich zur besseren Phasentrennung pH 1.

Die wäßrige Phase wurde abgetrennt und — um eine Phasentrennung bei der Extraktion mit Chlorbenzol zu ermöglichen — mit 2 l Wasser verdünnt. Nach der Verdünnung wurde zweimal mit je 250 ml = 278 g chlorbenzol extrahiert (Wasser oben).

Durch Fraktionierung der vereinigten organischen Phasen (3 116 g) über eine silberverspiegelte Vigreux-Kolonne, Trennhöhe 120 cm, Durchmesser innen 2,5 cm, wurden erhalten :

| Frakt. | Menge (g) | | Temp. (°C) Sumpf | Kopf | Druck (Torr) | Rücklauf | Gehalt in Flächen % GC[1] ClBz[2] | m-Kr.[3] | 3-PT[4] |
|---|---|---|---|---|---|---|---|---|---|
| 1 | 1 363 | org. | 110-195 | 91-132 | 760 | 1 : 1 | 98,6 | 1,1 | — |
| | + 51 | $H_2O$ | | | | | | | |
| 2 | 178 | | 100-110 | 46-92 | 35-9 | 1 : 1 | 56,2 | 43 | 0,6 |
| 3 | 744 | | 110-115 | 92-94 | 10 | 1 : 1 | | 98,2 | 1,8 |
| 4 | 66 | | 132-138 | 95-96 | 9 | 2 : 1 | | 95,7 | 4,3 |
| 5 | 43 | | 138-142 | 96-138 | 9 | 2 : 1 | | 63,9 | 36,1 |
| 6 | 567 | | 146-185 | 130-185 | 8-5,5 | 2 : 1 | | | 99,3 |
| 29 Rückstand | | | | | | | | | |
| 6 Kaltefalle | | | | | | | | | |
| 69 Verluste (Kolonnen-hold-up, Proben etc.) | | | | | | | | | |

[1]) Säulenlänge 2 m, Säulenmaterial SE30, Temperatur programmiert 70-280 °C.
[2]) Chlorbenzol.
[3]) m-Kresol.
[4]) 3-Phenoxytoluol.

Eine Ausbeuteberechnung, in der nur das in Fraktion 6 erhaltene reine m-Phenoxytoluol gemäß 567 g · 0,993 = 563 g → 3,06 Mol berücksichtigt wird und im übrigen mit Flächen-% = Gew.-% gerechnet wird, ergibt folgende isolierte Ausbeuten :

79,4 %, bezogen auf Chlorbenzol
85,7 %, bezogen auf m-Kresol
86,7 %, bezogen auf eingesetzte KOH (eine GC-analytische Bestimmung des 3-Phenoxytoluolproduktes in dem zur Destillation gelangenden Gemisch hatte eine praktisch 100 %ige Ausbeute ergeben).

Beispiel 3

3-Phenoxytoluol aus K-Phenolat und 3-Chlortoluol

In $N_2$-Atmosphäre wurde vorgelegt

63,75 g 88 %ige feste KOH → 1 Mol.

Dazu wurde unter Rühren eine Lösung von

282,3 g → 3 Mol Phenol in
379,7 g → 3 Mol 3-Chlortoluol gegeben.

Dabei stieg die Temperatur von ca. 20 °C auf ca. 40 °C an.

Danach wurde das Gemisch mittels eines Wasserauskreisers — der mit 83 g 3-Chlortoluol gefüllt worden war — in ca. 1 Stunde entwässert (ca. 27 ml wäßrige Phase). Temperatur im Kolben 123-162 °C.

Nach dem Abkühlen auf ca. 160 °C wurde zu der klaren Lösung 1 g $CuCO_3 \cdot Cu(OH)_2 \cdot 1/2\ H_2O$ (= 0,004 Mol) gegeben. Es bildete sich eine schwarze Lösung.

Das Gemisch wurde dann insgesamt 4 Stunden lang bei 168-170 °C am leichten Rückfluß erhitzt. Es entstand zunächst eine grünliche und zum Schluß eine rote Suspension. Am Ende dieser Periode befanden sich 64 g Flüssigkeit (78 Flächen-% 3-Chlortoluol, 18 Flächen-% Phenol) im Wasserauskreiser. Das Gewicht des Reaktionsgemisches betrug 705 g. Aus letzterem wurde nach Wäsche mit 55 g 3-Chlortoluol 94 g Lösungsmittelfeucht = 75,5 g trocken eines Salzgemisches über eine Nutsche abgesaugt. Das Gewicht der organischen Phase betrug 654 g (pH 6-7 nach Zusatz von Wasser). Gemäß GC-Analyse mit Dodecan als innerem Standard enthielt die organische Phase 28,5 Gew.-% 3-Phenoxytoluol → 186 g → 1,01 Mol, d. h. die auf eingesetztes KOH bezogene Ausbeute beträgt praktisch 100 %.

GC-Analysen während der Reaktion

| Stunden Rückfluß | 0 | 1 | 2 | 3 | 4 |
|---|---|---|---|---|---|
| Fl.-% 3-Phenoxytoluol | 0 | 23,7 | 28,0 | 28,1 | 28,0 |

Das heißt, die Reaktion ist nach 2 Stunden praktisch abgeschlossen.

## Beispiel 4

4-Phenoxytoluol aus K-Phenolat und 4-Chlortoluol

Es wurde wie in Beispiel 3 vorgegangen, wobei lediglich 3-Chlortoluol durch 4-Chlortolul ersetzt wurde.

Die Reaktion wurde bei nur sehr leichtem Rückfluß durchgeführt, da sonst Schäumen auftrat (166-167 °C). Während der Reaktion verfärbte sich das Gemisch von schwarz über violett in eine rotbraune Suspension (689 g) → 648 g Filtrat, nach Wäsche mit 55 g p-Chlortoluol verbleiben 84 g Filterkuchen, Lösungsmittel-feucht → 72 g trocken (pH-Wert der organischen Phase 7-8 nach Wasserzugabe). Gemäß GC-Analyse mit Dodecan als innerem Standard enthielt die organische Phase 28,1 Gew.-% 4-Phenoxytoluol → 182 g → 0,99 Mol, d. h., die auf eingesetztes KOH bezogene Ausbeute beträgt praktisch 100 %.

GC-Analysen während der Reaktion

| Stunden am Rückfluß | 0 | 1 | 2 | 3 | 4 | 5 | 6 |
|---|---|---|---|---|---|---|---|
| Fl.-% 4-Phenoxytoluol | — | 21,9 | 26,5 | 27,1 | 27,4 | 27,4 | 27,4 |

Das heißt, nach ca. 3 Stunden ist die Reaktion abgeschlossen.

## Beispiel 5

2-Phenoxytoluol aus K-Phenolat und o-Chlortoluol

In der Art von Beispiel 2 wurde aus 255 g 88 %iger KOH (→ 4 Mol), 1 519 g o-chlortoluol (→ 12 Mol) und 1 129 g Phenol (→ 12 Mol) eine Lösung von K-Phenolat hergestellt. Durch Zusatz von 4 g pulverförmigem $CuCO_3 \cdot Cu(OH)_2 \cdot 1/2\ H_2O$ (→ 0,017 Mol) wurde bei ca. 150 °C die Reaktion zwischen K-Phenolat und o-Chlortoluol eingeleitet. Sie wurde dann bei Rückflußtemperatur, 162 – 164 °C, 4 Stunden lang fortgeführt. Anhand von Proben wurde durch Titration mit Säure festgestellt, daß nach 2 Stunden 55 % und nach 4 Stunden 90 % des K-Phenolats angesetzt waren. Gemäß GC-Anlyse enthielt die Reaktionslösung nach 4 Stunden 650 g 2-Phenoxytoluol (→ 3,53 Mol), was einer Ausbeute von 88,3%-bezogen auf eingesetztes KOH bzw. einer Selektivität von 98 %, bezogen auf umgesetztes K-Phenolat, entspricht.

Aus dem Reaktionsgemisch wurden die Feststoffe abfiltriert und durch fraktionierte Destillation über eine 50 cm hohe Vigreux-Kolonne wurden bei $Kp_5$ 134-135 °C 620 g 2-Phenoxytoluol einer Reinheit von 98 % erhalten, was einer auf KOH bezogenen isolierten Ausbeute von 82,6 % entspricht.

Beispiel 6

4-Chlor-4'Methyl-Diphenyläther aus K-p-Kresolat und 1,4 Dichlorbenzol

Ein Gemisch aus

127,5 KOH 88 %ig → 2 Mol
649 g p-Kresol → 6 Mol
1 470 g 1,4-Dichlorbenzol → 10 Mol
1 000 g Xylol (als Schleppmittel bei der Entwässerung)

wurde in der Art von Beispiel 2 entwässert. Danach wurde die Reaktion durch Zugabe von 4 g $CuCO_3 \cdot Cu(OH)_2 \cdot 1/2\ H_2O$ bei ca. 130 °C eingeleitet und ca. 5 Stunden lang bei Rückflußtemperatur, 146-149 °C, fortgeführt. Danach waren laut acidimetrischer Titration 90 % des eingesetzten KOH umgesetzt und laut GC-Analyse enthielt das Reaktionsgemisch 355 g 4-Chlor-4'-Methyl-Diphenyläther, was einer Ausbeute von 81,2 %, bezogen auf eingesetzte KOH, bzw. einer selektivität von 90,3 %, bezogen auf umgesetztes K-p-Kresolat, entspricht.

Nach dem Abfiltrieren der Feststoffe wurden durch fraktionierte Destillation über eine 1,20 m hohe Vigreux-Kolonne 320 g 4-Chlor-4'-Methyl-Diphenyläther bei $Kp_6$ 154 °V in einer Reinheit von 98 % gewonnen, was einer auf KOH bezogenen isolierten Ausbeute von 71,8 % entspricht.

Beispiel 7

4-Brom-4'-Methyl-Diphenyläther und Hydrochinon-di-p-tolyläther aus K-p-Kresolat und 1,4-Dibrombenzol

Ein Gemisch aus

127,5 g KOH 88 %ig → 2 Mol
432,6 g p-Kresol → 4 Mol
1 916 g 1,4-Dibronbenzol → 8 Mol
1 050 g Xylol

wurde in der Art von Beispiel 2 entwässert. Danach wurden bei 95 °C 8 g $CuCO_3 \cdot Cu(OH)_2 \cdot 1/2\ H_2O$ (→ 0,034 Mol) zugegeben, dann wurde die Temperatur des Reaktionsgemisches wieder auf ca. 140 °C erhöht. Unter Temperatursteigerung durch die Reaktionswärme auf 160 °C und starken Siedeerscheinungen war dann die Reaktion nach wenigen Minuten abgeschlossen. Acidimetrisch wurde ein KOH-Umsatz von 98 % gefunden. Laut GC-Analyse enthielt das Gemisch 476 g 4-Brom-4'-Methyl-Diphenyläther, was einer auf KOH bezogenen Ausbeute von 90,5 % entspricht.

Nach dem Abfiltrieren der Feststoffe wurden durch fraktionierte Destillation über eine 1,20 m hohe Vigreux-Kolonne 450 g des Produktes bei $Kp_5$ 170 °C einer Reinheit von 97 % gewonnen, was einer isolierten Ausbeute von 83 % entspricht (bezogen auf KOH).

Das Hauptnebenprodukt der Reaktion ist Hydrochinon-di-p-tolyläther. In verstärktem Maße entsteht dieses Nebenprodukt, wenn der Überschuß von 1,4-Dibrombenzol verkleinert wird bzw. wenn ein Überschuß an K-p-Kresolat gegenüber 1,4-Dibrombenzol benutzt wird.

Setzt man beispielsweise in der Art dieses Beispiels ein Gemisch aus

255 g 88 %ige KOH → 4 Mol
1 298 g p-Kresol → 12 Mol
930 g 1,4 Dibrombenzol → 3,95 Mol
1 000 g Xylol

nach der Entwässerung durch Zusatz von 4 g $CuCO_3 \cdot Cu(OH)_3 \cdot 1/2\ H_2O$ (→ 0,017 Mol) um, so entstehen laut GC-Analyse nach der spontanen Reaktion 720 g 4-Brom-4'-Methyl-Diphenyläther (→ 2,74 Mol) 180 g Hydrochinon-di-p-tolyläther (→ 0,62 Mol), d. h. das K-p-Kresolat reagiert zu ca. 70 % mit einem Brom- und zu ca. 30 % mit beiden Bromatomen am 1,4-Dibrombenzol.

Hydrochinon-di-p-tolyläther reichert sich bei der fraktionierten Destillation im Kolonnensumpf im hohem Maße an, und kann daraus durch Umkristallisation in Äthanol oder Toluol/Äthanol in reiner Form, F. 103 °C, gewonnen werden.

Beispiel 8

4-Fluor-4'-methyldiphenylether aus p-Fluorphenol und p-Chlortoluol

Ein Gemisch aus

175 g 4-Fluorphenol → 1,56 Mol
197,6 g 4-Chlortoluol → 1,36 Mol
33,3 g KOH 88 %ig → 0,52 Mol

wurde in $N_2$-Atmosphäre bei Normaldruck entwässert. Dabei wurde unter nur schwachem Rückfluß gearbeitet, um Behinderungen durch Schaumbildung zu unterdrücken. Während der Entwässerung stieg die Temperatur im Reaktionskolben von 142 auf 162 °C an und es trat eine Farbveränderung von gelblich nach rotbraun ein.

Nach der Entwässerung wurde bei ca. 150 °C 1 g $CuCO_3 \cdot Cu(OH)_2 \cdot 1/2\ H_2O$ zugegeben und anschließend 1 Stunde lang am Rückfluß (162 °C) erhitzt. Die acidimetrische Verfolgung der Reaktion zeigte, daß sie bereits nach 0,5 Stunden praktisch beendet war:

| | |
|---|---|
| vor Katalysator-Zugabe | 0,52 Mol KOH |
| 1/2 Std. später | 0,047 Mol KOH |
| 1 Std. später | 0,027 Mol KOH |

Das Reaktionsgemisch wurde auf Raumtemperatur abgekühlt, der Feststoff über eine Nutsche abgesaugt und mit 3 × 50 ml p-Chlortoluol gewaschen. Es fielen 52 g Filterkuchen-Lösungsmittel feucht bzw. 44 g Filterkuchen trocken an (theor. 38,9 g → 0,52 Mol KCl; $Cl^-$ 31,7 %, $F^-$ 2,9 %). Aus der vorgenannten Analyse geht hervor, daß kernständiges Fluor während der Reaktion nur in untergeordnetem Maße substituiert wurde.

Das Filtrat (502 g) wurde über eine 30 cm hohe, mit 3 mm Braunschweiger Glaswendeln gefüllte Kolonne fraktioniert. Bei $Kp._7$ 138 °C, wurden 60 g 4-Fluor-4'-methyldiphenylether in 98 %iger Reinheit als klare Flüssigkeit gewonnen, was einer isolierten Ausbeute von 57,1 %, bezogen auf den KOH-Einsatz entspricht. Unumgesetztes 4-Fluorphenol bzw. 4-Chlortoluol wurde bei dieser Fraktionierung als Vorlauf abgetrennt.

**Ansprüche**

1. Verfahren zur Herstellung von Diphenylethern durch Umsetzung von unsubstituierten oder durch niedere Alkylgruppen, Halogen, CN, CHO und/oder COOR (R = niederes Alkyl) ein- oder mehrfach substituierten Alkaliphenolaten mit unsubstituierten oder durch die gleichen wie die bei den Alkaliphenolaten genannten Substituenten ein- oder mehrfach substituierten Halogenbenzolen in Gegenwart von Cu-Verbindungen als Katalysatoren bei erhöhter Temperatur, dadurch gekennzeichnet, daß man als Cu-Verbindungen basische Cu-Carbonate der Formeln

$$2\ CuCO_3 \cdot Cu(OH)_2,\ CuCO_3 \cdot Cu(OH)_2 \cdot H_2O \text{ und/oder } CuCO_3 \cdot Cu(OH)_2 \cdot 1/2\ H_2O$$

verwendet.

2. Verfahren nach Anspruch 1, dadurch gekennzeichnet, daß man als basisches Cu-Carbonat die Verbindung der ungefähren Zusammensetzung $CuCO_3 \cdot Cu(OH)_2 \cdot 1/2\ H_2O$ verwendet.

3. Verfahren nach den Ansprüchen 1 bis 2, dadurch gekennzeichnet, daß man die Cu-Katalysatoren in einer Menge von 0,000 1 bis 5, vorzugsweise 0,001 bis 0,1 Mol-%, bezogen auf das Alkaliphenolat, verwendet.

4. Verfahren nach den Ansprüchen 1 bis 3, dadurch gekennzeichnet, daß man als Alkaliphenolate nicht weiter substituiertes oder durch eine niedere Alkylgruppe oder Fluor einfach substituiertes Alkaliphenolat verwendet.

5. Verfahren nach den Ansprüchen 1 bis 4, dadurch gekennzeichnet, daß man als Halogenbenzole Mono- und Dichlorbenzole, Mono- und Dibrombenzole oder durch eine niedere Alkylgruppe einfach substituiertes Monochlor- oder Monobrombenzol, vorzugsweise jeweils nur die Cl-Verbindungen, verwendet.

6. Verfahren nach den Ansprüchen 1 bis 5, dadurch gekennzeichnet, daß man als Halogenphenole nicht weiter substituiertes oder durch eine niedere Alkylgruppe einfach substituiertes Monochlor- oder Monobrombenzol, vorzugsweise Monochlorbenzol, verwendet.

7. Verfahren nach den Ansprüchen 1 bis 6, dadurch gekennzeichnet, daß man einen der Reaktionspartner Alkaliphenolat und Halogenbenzol in nicht weiter substituierter und den anderen in durch eine niedere Alkylgruppe einfach substituierter Form verwendet.

8. Verfahren nach den Ansprüchen 1 bis 7, dadurch gekennzeichnet, daß man die Umsetzung bei einer Temperatur zwischen 100 und 200 °C, vorzugsweise zwischen 130 und 170 °C, durchführt.

9. Verfahren nach den Ansprüchen 1 bis 8, dadurch gekennzeichnet, daß man die Umsetzung in Gegenwart von überschüssigem, dem eingesetzten Alkaliphenolat zugrundeliegenden freien Phenol durchführt.

**Claims**

1. A process for the preparation of diphenyl ethers by reaction of alkali metal phenolates either unsubstituted or mono- or polysubstituted by lower alkyl, halogen, CN, CHO, COOR (R = lower alkyl) and/or with halobenzenes either unsubstituted or mono- or polysubstituted by the same substituents as mentioned before and in the presence of copper compounds as catalysts at elevated temperature, characterized in using as copper compounds basic copper carbonates of the formulae

$$2\ CuCO_3 \cdot CU(OH)_2,\ CuCO_3 \cdot Cu(OH)_2 \cdot H_2O \text{ and/or } CuCO_3 \cdot Cu(OH)_2 \cdot 1/2\ H_2O.$$

2. The process as claimed in claim 1, characterized in using as basic copper carbonate the compound having approximately the following composition : $CuCO_2 \cdot Cu(OH)_2 \cdot 1/2\ H_2O$.

3. The process as claimed in claims 1 to 2, characterized in using the copper catalysts in an amount of from 0.000 1 to 5, preferably 0.001 to 0.1, mol-%, relative to the alkali metal phenolate.

4. The process as claimed in claims 1 to 3, characterized in using as alkali metal phenolates an alkali metal phenolate either unsubstituted or monosubstituted by lower alkyl or fluorine.

5. The process as claimed in claims 1 to 4, characterized in using as halobenzenes mono- or dichlorobenzenes, mono- or dibromobenzenes, or monochlor- or monobromobenzene monosubstituted by lower alkyl, preferably the Cl compounds only.

6. The process as claimed in claims 1 to 5, characterized in using as halophenols monochlor- or monobromobenzene either unsubstituted or monosubstituted by lower alkyl, preferably only monochlorobenzene.

7. The process as claimed in claims 1 to 6, characterized in using one of the reactants alkali metal phenolate and halobenzene in unsubstituted form, and the other as compound monosubstituted by lower alkyl.

8. The process as claimed in claims 1 to 7, characterized in carrying out the reaction at a temperature of from 100 to 200 °C, preferably 130 to 170 °C.

9. The process as claimed in claims 1 to 8, characterized in carrying out the reaction in the presence of an excess of the free phenol on which the alkali metal phenolate used is based.

**Revendications**

1. Procédé de préparation d'oxydes de diphényles par réaction de phénolates alcalins non substitués ou substitués, une ou plusieurs fois, par des groupes alkyles inférieurs, par de l'halogène, par CN, par CHO et/ou COOR (où R est un groupe alkyle inférieur) avec des halogénobenzènes non substitués, ou substitués une ou plusieurs fois par les mêmes substituants que ceux cités pour les phénolates alcalins, en présence de composés du cuivre comme catalyseurs, à température élevée, procédé caractérisé en ce qu'on utilise comme composés de Cu des carbonates basiques de Cu répondant aux formules

$$2\ CuCO_3 \cdot Cu(OH)_2,\ CuCO_3 \cdot Cu(OH)_2 \cdot H_2O \text{ et/ou } CuCO_3 \cdot Cu(OH)_2 \cdot 1/2\ H_2O$$

2. Procédé selon la revendication 1, caractérisé en ce qu'on utilise comme carbonate basique de Cu le composé ayant comme composition approximative $CuCO_3 \cdot Cu(OH)_2 \cdot 1/2\ H_2O$.

3. Procédé selon les revendications 1 et 2, caractérisé en ce qu'on utilise les catalyseurs à base de cuivre en une quantité de 0,000 1 à 5, de préférence 0,001 à 0,1 mol-%, par rapport au phénolate alcalin.

4. Procédé selon les revendications 1 à 3, caractérisé en ce qu'on utilise comme phénolates alcalins du phénolate alcalin qui n'est pas davantage substitué ou qui est substitué une fois par un groupe alkyle inférieur ou par du fluor.

5. Procédé selon les revendications 1 à 4, caractérisé en ce qu'on utilise comme halogénobenzènes des mono- et dichlorobenzènes, des mono- et dibromobenzènes ou du mono- chloro- ou monobromobenzène substitué une fois par un groupe alkyle inférieur, mais de préférence seulement les composés chlorés.

6. Procédé selon les revendications 1 à 5, caractérisé en ce qu'on utilise comme halogénophénols du monochloro- ou monobromophénol qui n'est pas substitué davantage ou qui est substitué une fois par un groupe alkyle inférieur, de préférence du monochlorobenzène.

7. Procédé selon les revendications 1 à 6, caractérisé en ce qu'on utilise l'un des corps participant à

la réaction, le phénolate alcalin et l'halogénobenzène, sous une forme qui n'est pas davantage substituée et l'autre corps sous une forme substituée une fois par un groupe alkyle inférieur.

8. Procédé selon les revendications 1 à 7, caractérisé en ce qu'on effectue la réaction à une température comprise entre 100 et 200 °C, de préférence entre 130 et 170 °C.

9. Procédé selon les revendications 1 à 8, caractérisé en ce qu'on effectue la réaction en présence d'un excès du phénol libre correspondant au phénolate alcalin mis en œuvre.